(19) Europäisches Patentamt
European Patent Office
Office européen des brevets

(11) EP 0 232 295 B2

(12) # NEUE EUROPÄISCHE PATENTSCHRIFT

(45) Veröffentlichungstag und Bekanntmachung des Hinweises auf die Entscheidung über den Einspruch:
**04.08.1999 Patentblatt 1999/31**

(45) Hinweis auf die Patenterteilung:
**16.06.1993 Patentblatt 1993/24**

(21) Anmeldenummer: **86904109.5**

(22) Anmeldetag: **21.07.1986**

(51) Int. Cl.$^6$: **C07D 498/20**, C07D 498/22, G03C 1/72
// (C07D498/20, 265:00, 221:00, 209:00), (C07D498/22, 265:00)

(86) Internationale Anmeldenummer:
**PCT/DE86/00300**

(87) Internationale Veröffentlichungsnummer:
**WO 87/00524 (29.01.1987 Gazette 1987/03)**

(54) **PHOTOCHROME VERBINDUNGEN**
PHOTOCHROMIC COMPOUNDS
COMPOSES PHOTOCHROMIQUES

(84) Benannte Vertragsstaaten:
**AT DE FR GB IT**

(30) Priorität: **19.07.1985 DE 3525891**

(43) Veröffentlichungstag der Anmeldung:
**19.08.1987 Patentblatt 1987/34**

(73) Patentinhaber:
**Optische Werke G. Rodenstock**
**80469 München (DE)**

(72) Erfinder: **MELZIG, Manfred**
**D-8031 Wessling (DE)**

(74) Vertreter:
**Schiller, Walter, Dr. et al**
**Kanzlei Münich, Steinmann, Schiller,**
**Wilhelm-Mayr-Str. 11**
**80689 München (DE)**

(56) Entgegenhaltungen:
**EP-A- 0 141 407**

Bemerkungen:
Die Akte enthält technische Angaben, die nach dem Eingang der Anmeldung eingereicht wurden und die nicht in dieser Patentschrift enthalten sind.

EP 0 232 295 B2

## Beschreibung

### Technisches Gebiet

Die Erfindung bezieht sich auf photochrome Verbindungen, d.h. auf Substanzen, deren Absorptionseigenschaften sich entsprechend der Umgebungsbeleuchtung ändern, so daß sich auch die Einfärbung von mit diesen Substanzen gefärbten Materialien entsprechend der Umgebungsbeleuchtung in Intensität und Farbeindruck ändert.

Photochrome Substanzen haben eine Vielzahl von Anwendungsmöglichkeiten, beispielsweise werden sie zur Herstellung von photochromen Sonnenschutz-Brillengläsern aus Kunststoff, von Brillenfassungen etc. verwendet.

### Stand der Technik

Photochrome Substanzen sind beispielsweise aus den US-PSen 3 562 172, 3 578 602, 4 215 010 oder der DE-OS 29 26 255 bekannt.

Die aus diesen Druckschriften bekannten photochromen Substanzen bzw. Verbindungen haben jedoch unter anderem den Nachteil, daß der photochrome Effekt stark temperaturabhängig ist: Mit steigenden Umgebungstemperaturen wird die Eindunkelung bei gleicher Beleuchtungsstärke geringer.

Deshalb sind in der WO 85/02619 photochrome Substanzen vorgeschlagen worden, deren allgemeines Gerüst mit dem der in den vorstehend genannten Druckschriften beschriebenen Substanzen übereinstimmt, bei denen jedoch durch geeignete an dem Gerüst angelagerte Reste die Temperaturabhängigkeit des photochromen Effekts wesentlich verringert wird.

Die aus der WO 85/02619 bekannten Substanzen haben jedoch teilweise den Nachteil, daß sie auch im nicht bestrahlten Zustand eine leicht bläuliche Einfärbung aufweisen. Ein mit diesen Substanzen eingefärbtes Brillenglas hat deshalb auch im nicht bestrahlten Zustand eine leicht bläuliche Färbung, durch die die Transmission $\tau(V_\lambda)$ im nicht bestrahlten Zustand unter Umständen kleiner als 85%, dem beispielsweise für KFZ-Lenker für Nachtfahrten empfohlenen Wert ist.

Auch mit den aus der DDR-PS 156 372 bekannten ähnlich aufgebauten 1,3,3-Trimethylindolinspirophenanthro-1.4-2H-oxazinen können nur stark violett getönte Gläser hergestellt werden. Dies gilt bereits ohne Substituenten im Phenanthrensystem. Durch Einführung ziehender Substituenten (2- oder 4-Nitrophenanthren) ergeben sich sogar noch stärkere Tönungen im nicht bestrahlten Zustand. Das 2, 7-Dinitroderivat schließlich liegt schon bei Raumtemperatur fast vollständig in der farbigen Form vor.

Deshalb sind in der EU-OS 0141 407 photochrome Substanzen vorgeschlagen worden, die einen Aufbau gemäß der im Patentanspruch 1 angegebenen allgemeinen Formel haben. Diese phototropen Substanzen sind im nicht angeregten Zustand farblos, d.h. sie vermindern die Transmission beispielsweise eines Brillenglases praktisch nicht. Nachteilig bei den aus dieser Druckschritt bekannten phototropen Verbindungen ist es jedoch, daß sie in üblicherweise für Kunststoff-Brillengläsern eingesetzten Materialien, wie beispielsweise Diethylenglykol-bis-allylcarbonat eine vergleichsweise große Neigung zur Migration zeigen. Dies ist beim Entspiegeln der Brillenglaser von Nachteil, da die im Vakuum bei erhöhter Temperatur an die Oberfläche wandernden phototropen Farbstoffmoleküle nicht haftende Entspiegelungsschichten ergeben.

### Darstellung der Erfindung

Der Erfindung liegt die Aufgabe zugrunde. photochrome Verbindungen anzugeben, die lediglich eine geringe Neigung zur Migration zeigen, so daß Aufdampfschichten gut haften, und weiterhin im nicht bestrahlten Zustand eine vergleichsweise geringe Einfärbung und eine nur geringe Temperaturabhängigkeit des photochromen Effekts aufweisen.

Diese Aufgabe wird erfindungsgemäß durch Verbindungen mit der allgemeinen Formel

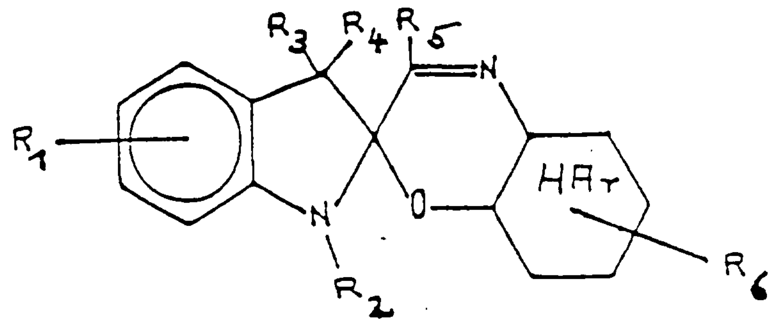

( 1 )

gelöst, worin die Bezeichnungen bedeuten:

| | |
|---|---|
| $R_1$ | einen bis vier Substituenten aus der Reihe -Z, -OZ, $-NZ_2$, -NHZ, $-NH_2$, -CN, $-CF_3$ mit Z: $C_1$-$C_5$ Alkyl, Phenyl, Pyridyl, Benzyl |
| $R_2$ | -H, -Z, $-CH_2Z$, |
| $R_3,R_4,R_5$ | $-NH_2$, NHZ, $-NZ_2$, -OZ, -Z, -H |
| $R_6$ | -H, -Z, -OZ, -CN, $-NO_2$ |
| HAr | zwei- oder dreikerniger Heteroaromaten mit 1 oder 2 N-Atomen gemäß einer der nachstehenden Strukturformeln |

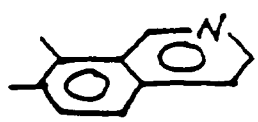

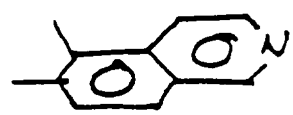

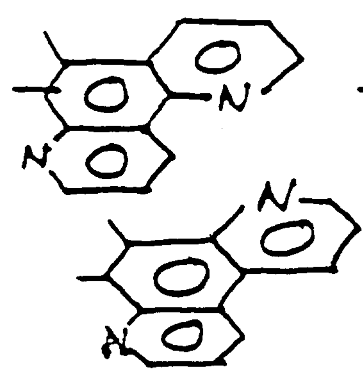

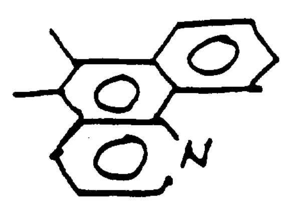

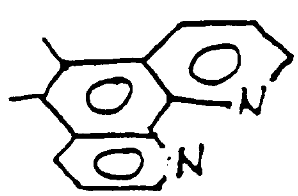

Erfindungsgemäß ist erkannt worden, daß die Migration der phototropen Moleküle durch die Verwendung anderer Heteroaromaten (HAr) als in der EU-OS 0 141 407 beschrieben, wesentlich verringert wird. Die erfindungsgemäß verwendeten Heteroaromaten und insbesondere die bevorzugt verwendete Benzo- oder Pyridoannelierung sind wesentlich vorteilhafter als längere C-Ketten am Indolin-Stickstoffatom, wie sie gemäß der EU-OS 0 141 407 verwendet werden, da diese Verbindungen dem phototropen Molekül bereits Weichmachereigenschaften verleihen.

Damit haben die erfindungsgemäß vorgeschlagenen Verbindungen gegenüber den bekannten Molekülen den

Vorteil, daß ihre Migration geringer ist, so daß mit ihnen dotierte Kunststoffgläser ohne weiteres entspiegelt werden können.

Für die Verringerung der Temperaturabhängigkeit des phototropen Effekts sind weiterhin, wie bereits in der WO 85/02619 beschrieben, die Reste $R_2$, $R_3$, $R_4$ und $R_5$ entscheidend: Durch die Einwirkung von kurzwelligem Licht öffnet sich die Spiro-C-O-Bindung; hierbei entstehen aus der Grundform, d.h. dem Molekül mit der allgemeinen Formel (1) folgende Moleküle, die miteinander im Gleichgewicht stehen:

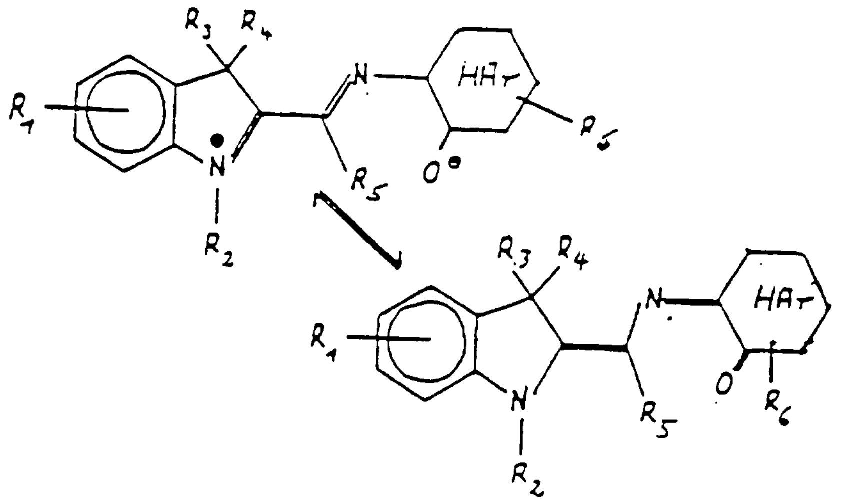

(2)

Die Moleküle mit der Strukturformel (2) haben eine blaue Farbe, während das Molekül mit der Strukturformel (1) farblos ist.

Die erfindungsgemäßen Verbindungen mit der allgemeinen Formel (1) weisen anstelle eines Naphtalinrestes bei den aus der WO 85/02619 bekannten Verbindungen bzw. eines Phenanthren-Restes gemäß der DDR-PS156 372 einen zwei-oder dreikernigen Heteroaromaten mit 1 oder 2 N-Atomen (0 bis 2 N-Atome pro Kern) auf. Aufgrund dieser Modifikation des "Gerüsts" der Spiroverbindungen, die durch die EU-OS 0141 407 nicht nahegelegt ist, und auf deren Vorteile hinsichtlich der Migrationseigenschaften sich auch kein Hinweis im Stand der Technik findet (s. beispielsweise den in eine andere Richtung gehenden Lösungsversuch bei den aus der DDR-PS 156 372 bekannten Molekülen), erhält man auch bei Molekülen mit geringer Migration ein Gleichgewicht zwischen den Formen mit der allgemeinen Formel (2) und der Grundform (1), das im nicht bestrahlten Zustand näher bei der Grundform liegt, die farblos ist, so daß die Transmission der erfindungsgemäßen Verbindungen weiterhin im nicht bestrahlten Zustand größer ist als bei Molekülen mit vergleichbaren Migrationseigenschaften.

Auch bei den neu vorgeschlagenen Verbindungen mit der allgemeinen Formel (1) wird durch steigende Temperaturen die Rückreaktion begünstigt, d.h. das Gleichgewicht zwischen Molekülen mit der Strukturformel (1) und (2) wird bei gleicher Beleuchtungsstärke durch steigende Temperatur von der Modifikation (2) zu der Grundform (1) hin verschoben.

Der Rest $R_5$ stellt dabei eine starke sterische und zusätzlich eine elektronische Hinderung für die Rückreaktion dar. Dies trifft in etwas geringerem Maße auch für die Reste $R_2$, $R_3$ und $R_4$ zu.

In gleicher Weise wie die in der WO 85/02619 beschriebenen Verbindungen weisen die erfindungsgemäßen photochromen Substanzen den zusätzlichen Vorteil der positiven Thermochromie auf, wenn die Reste $R_3$ und $R_4$ größer als Methyl sind: Durch die positive Thermochromie wird mit steigender Temperatur eine beleuchtungsabhängige Eindunkelung hervorgerufen, die die negative Wirkung der Rückreaktion zum Teil wieder aufgehebt.

Weiterbildungen der Erfindung sind in den Unteransprüchen angegeben.

In den Ansprüchen 2 und 3 sind besonders bevorzugte erfindungsgemäße Verbindungen angegeben. die den Vorteil haben, daß sich die verwendeten Heteroaromaten nach bekannten Vorschriften synthetisieren lassen.

Die im Anspruch 4 beanspruchte Modifikation, bei der am Heteroaromatkern in Orthostellung zum N-Atom ein $CH_3$-Rest vorgesehen ist, eröffnet die Möglichkeit durch Substitution des $CH_3$-Rests beispielsweise durch

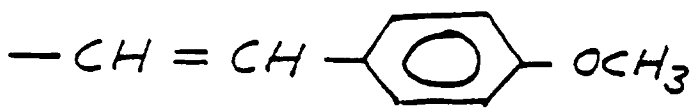

mittels Kondensation mit para-Methoxybenzalldehyd (Anspruch 5) stark farbige Ausgangsverbindungen herzustellen, deren Farbe im unbestrahlten Zustand z.B. orange bis rot ist, und die damit einen anderen Farbumschagseindruck vermitteln.

Im folgenden sollen erfindungsgemäße Verbindungen exemplarisch angegeben und mit photochromen Verbindungen nach dem Stand der Technik verglichen werden:

Die Synthese der erfindungsgemäßen photochromen Verbindung erfolgt in an sich bekannter Weise durch Kondensation eines substituierten 2-Alkylenindolins

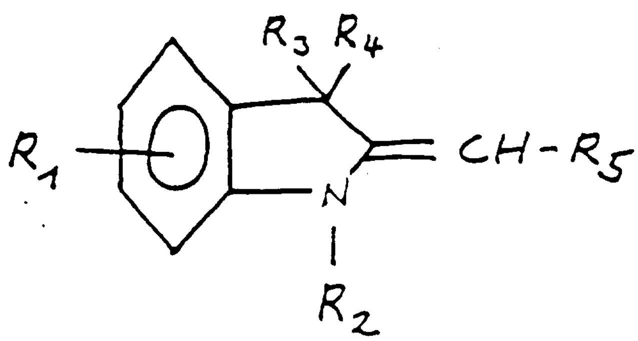

mit einem O-Hydroxy-nitroso-hetero-aromaten, beispielsweise mit 7-Hydroxy-8-Nitroso-isochinolin:

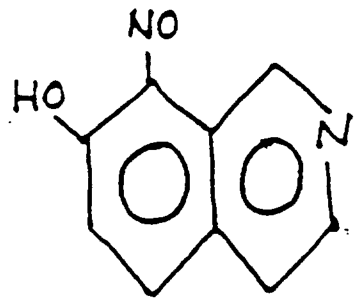

Die Bildung des 2-Alkylenindolins kann durch vorhergehende Synthese oder während des letzten Syntheseschrittes aus einem entsprechenden Indoleninium-Salz erfolgen. Ebenso ist die Einführung des Restes $R_5$ in diesem Schritt über das Kondensationsmittel möglich. Da die Reaktionsschritte im Prinzip bekannt sind, ist ein näheres Eingehen an dieser Stelle nicht erforderlich. Lediglich der letzte Reaktionsschritt soll kurz angedeutet werden:

In einem 250 ml Dreihalskolben, der mit einem Thermometer, einem Tropftrichter und einer Destillationsbrücke mit Reflux-Kühler und Wasserabscheider versehen ist, wird unter Rühren 0,01 mol einer o-Hydroxynitroso-Verbindung in 100 ml absolutem Ethanol suspendiert und zum Sieden erhitzt. Als Schlepper werden 5 ml Benzol zugesetzt. In die siedende Mischung werden 0,01 mol Indolinbase, gelöst in 50 ml absolutem Ethanol unter Zusatz von 0.9 ml Piperidin zugetropft. Die Raktionsmischung wird zwei Stunden am Sieden gehalten und anschließend das entstandene Reaktionswasser am Wasserabscheider abgetrennt.

Der erkalteten Lösung werden im Vakuum die Lösemittel entzogen. Der dunkelbraune, oftmals teerige Rückstand wird mit Methylenchlorid an Aluminiumoxid chromatographiert. Die Hauptfraktion wird im Vakuum zum Trocknen eingedampft und anschließend aus Methanol umkristallisiert.

Wird die Kristallisation unter Dunkelheit und bei tiefen Temperaturen ausgeführt, so erhält man ein Pulver mit fahlgelber bis brauner Farbe mit Ausnahme der Fälle, in denen $R_2 = R_3 = C_2H_5$ und $R_2 = C_2H_5$ und $R_3 = C_3H_7$ ist. In

diesen Fällen ist die Farbe grün bis dunkelgrün. Bei Beleuchtung werden die Substanzen in der Regel mehr oder weniger grün entsprechend dem Anteil der Moleküle, die in die geöffnete blaue Form (Strukturformel 2) übergehen.

Mit den vorstehend beschriebenen Reaktionsschritten sind u.a. folgende Verbindungen hergestellt worden:

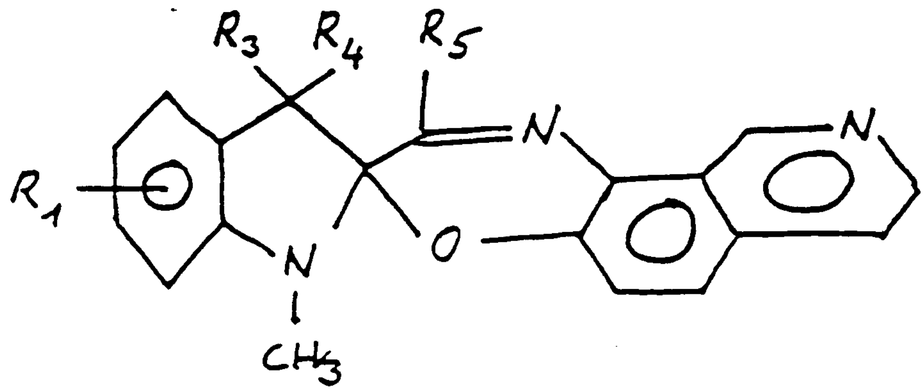

| Nr | $R_1$ | $R_3,R_4$ | $R_5$ | |
|---|---|---|---|---|
| 1 | -H | $-CH_3$ | -H | 1,3,3-Trimethyl-spiro(indolin-2,3' (3H)-pyrido(3,4-f)(1,4)benzoxazin) |
| 2 | -H | $-C_2H_5$ | -H | 3,3-Diethyl-1-methyl-spiro(indolin-2,3' (3H)-pyrido(3,4-f)(1,4)benzoxazin) |
| 3 | $-5-NH_2$ | $-CH_3$ | -H | 5-Amino-1,3,3-trimethyl-spiro(indolin-2,3' (3H)-pyrido(3,4-f)(1,4)benzoxazin) |

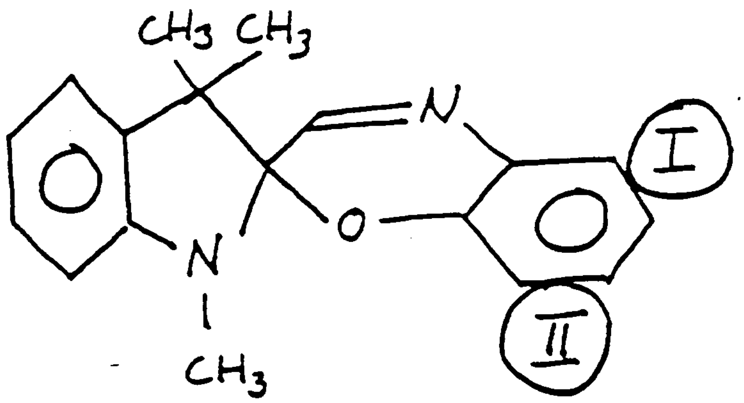

| Nr | I | II | |
|---|---|---|---|
| 4 | Benzo | Pyrido | 1,3,3-Trimethyl-spiro(indolin-2,3' (3H)-benzo(f)-pyrido(2,3-d)(1,4) benzoxazin |
| 5 | Pyrido | Pyrido | 1,3,3-Trimethyl-spiro(indolin-2,3' (3H)-dipyrido(3,2-d:3,2-f)(1,4) benzoxazin |

Nachstehend werden die NMR-Daten der erfindungsgemäßen Verbindungen 1 bis 5 angegeben. Mit Ausnahme der Verbindung 2, die in Aceton-d6 gelöst worden ist, sind sämtliche Verbindungen hierbei in $CDCl_3$ gelöst.

| Verbindung | 1 | 2 | 3 | 4 | 5 |
|---|---|---|---|---|---|
| D, 1H | 9,1 | 9,1 | 9.1 | | |
| M, 1H | 8,8 | 8,8 | 8,8 | | 9,0 |
| M, 2H | | | | | |
| M, 3H | | | | 9,1-8,6 | |
| M, 4H | | 1,85 | | | |
| M, 6H | | | | 7,7-6,2 | |
| M, 7H | | | 7,8-6,2 | | |
| M, 8H | 7,9-6,2 | 8,0-6,4 | | | 8,0-6,2 |
| QD, 1H | | | | | 9,4 |
| QS, 3H | | | | 7,9 | |
| Sbreit, 2H | | | 3,35 | | |
| S, 3H | 2,75 | 2,60 | 2,70 | 2,75 | 2,80 |
| S, 6H | 1,35 | | 1,35 | 1,35 | 1,35 |
| T, 3H | | 0,70 | | | |
| T, 3H | | 0,85 | | | |

Die Verbindungen 1 bis 5 zeigen ein weitgehend gleiches Verhalten bezüglich des phototropen Effekts, insbesondere der Transmission im unbestrahlten Zustand, Thermochromie, sowie der Migrationseigenschaften in Kunststoffmaterialien, wie sie für Brillengläser verwendet werden. Deshalb soll im folgenden lediglich die Verbindung 1 mit photochromen Verbindungen nach dem Stand der Technik verglichen werden, wie er aus der US-PS 4 215 010 und WO 85/02619 bekannt ist.

Die betrachteten Verbindungen nach dem Stand der Technik haben die allgemeine Formel im nicht bestrahlten Zustand:

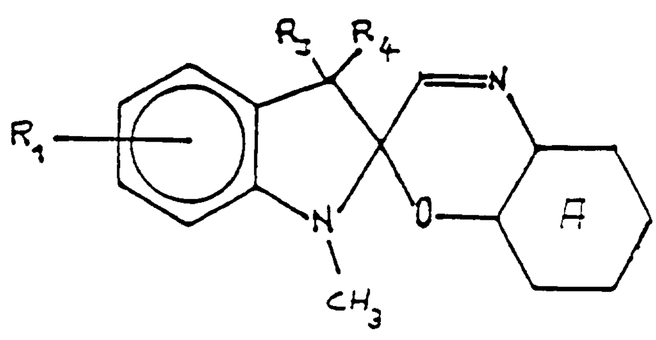

bei der aus der US-PS 4 215 010 bekannten Verbindung bedeutet:

A =

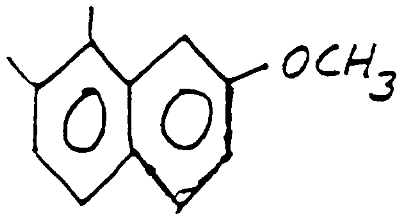

$R_3$, $R_4$ = $CH_3$,
während bei der aus der WO 85 02619 bekannten Verbindung bedeuten:
A =

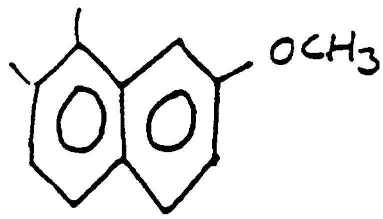

$R_3$, $R_4$ = $C_2H_5$,
In der nachstehenden Tabelle sind für die drei Verbindungen die Transmission $\tau_o(V_\lambda)$ im nicht bestrahlten Zustand bei 15°C sowie die Temperaturabhängigkeit der optischen Dichteänderung $\Delta$OD für eine Beleuchtungsstärke von 60 000 Lux angegeben. Die optische Dichteänderung ist dabei als $\Delta OD = \log\tau_o - \log\tau_s$ definiert, wobei $\tau_s$ die Transmission im bestrahlten Zustand und $\tau_o$ die Transmission im unbestrahlten Zustand ist.

| | Verb 1 | US-PS 4215010 | WO 85/02619 |
|---|---|---|---|
| $\tau_o(V_\lambda)$ | 91% | 92% | 85% |
| Temp (°C) | $\Delta$OD | $\Delta$OD | $\Delta$OD |
| 5 | 0,718 | 0,964 | |
| 15 | 0,584 | 0,602 | |
| 23 | 0,432 | 0,273 | 0,475 |
| 30 | 0,262 | 0,172 | 0,291 |
| 40 | 0,128 | 0,077 | |

Der vorstehenden Tabelle ist zu entnehmen, daß bei praktisch gleicher photochromer Leistung bei 15°C die erfindungsgemäße Verbindung gegenüber der aus der US-PS 421010 bekannten Verbindung eine geringere Eindunkelung bei niedrigeren Temperaturen und eine stärkere Eindunkelung bei höheren Temperaturen zeigt.

Im Vergleich zu der aus der WO 85/02619 bekannten Verbindung zeigt die erfindungsgemäße Verbindung (Verb 1) bei praktisch gleichem Temperaturverhalten der Eindunkelung eine deutlich größere Transmission im nicht bestrahlten Zustand, so daß mit den erfindungsgemäßen Verbindungen eingefärbte Brillengläser beispielsweise auch bei Nachtfahrten getragen werden können.

**Patentansprüche**

1. Verbindung der allgemeinen Formel

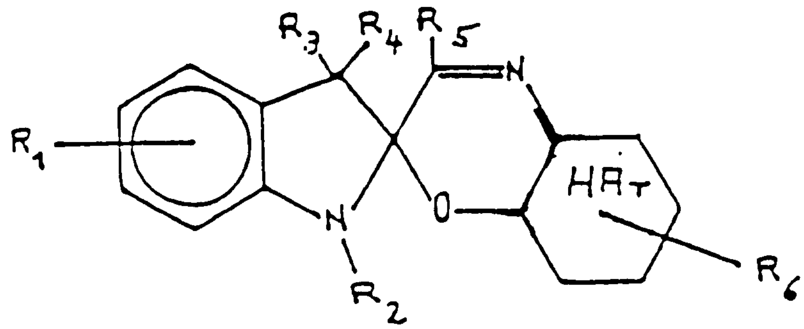

worin die Bezeichnungen bedeuten:

| | |
|---|---|
| $R_1$ | einen bis vier Substituenten aus der Reihe -Z, -OZ, $-NZ_2$, -NHZ, $-NH_2$, -CN, $-CF_3$ mit Z: $C_1$-$C_5$ Alkyl, Phenyl, Pyridyl, Benzyl |
| $R_2$ | -H, -Z, $-CH_2Z$, |
| $R_3$,$R_4$,$R_5$ | $-NH_2$, NHZ, $-NZ_2$, -OZ, -Z, -H |
| $R_6$ | -H, -Z, -OZ, -CN, $-NO_2$ |
| HAr | zwei- oder dreikerniger Heteroaromaten mit 1 oder 2 N-Atomen gemäß einer der nachstehenden Strukturformeln |

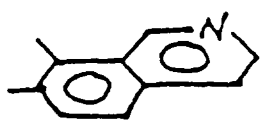

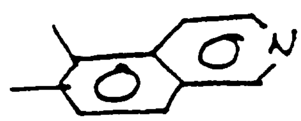

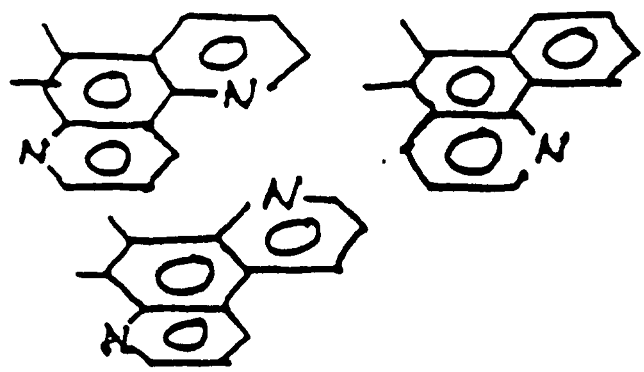

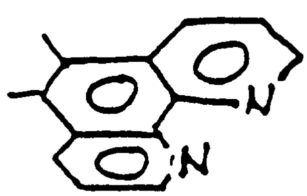

**2.** Verbindung der in Anspruch 1 angegebenen allgemeinen Formel mit

$R_1$: H,
$R_2$: $CH_3$

$R_3$,$R_4$: $CH_3$ oder $C_2H_5$
$R_5$: H

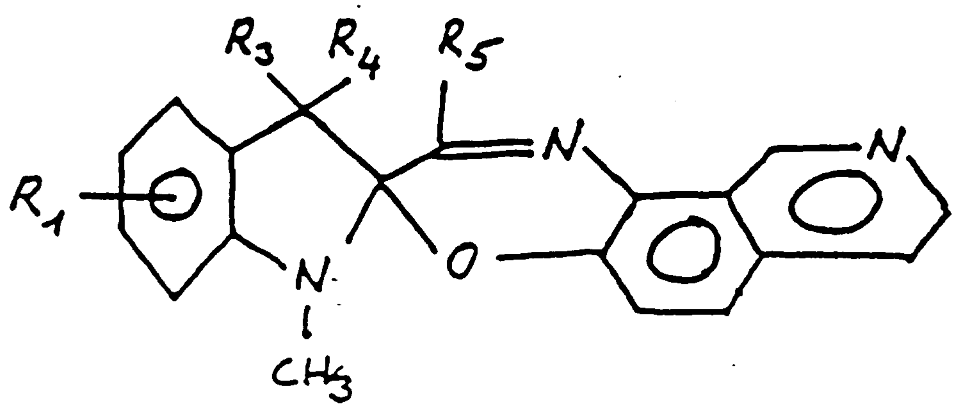

3. Verbindung der allgemeinen Formel nach Anspruch 1 mit

$R_1$,$R_5$: H,
$R_2$,$R_3$,$R_4$: $CH_3$

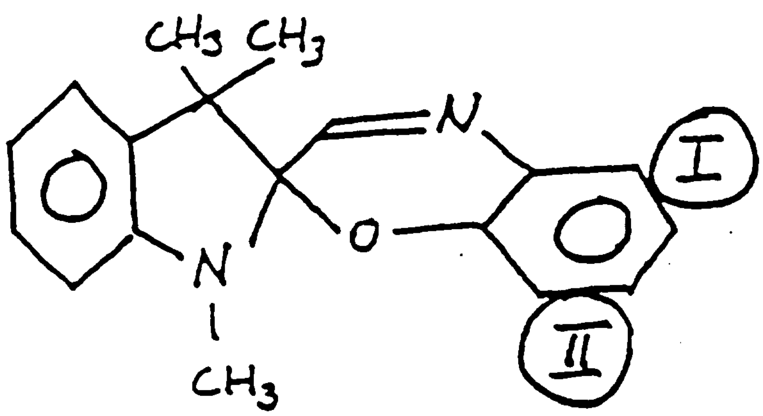

I: Benzo oder Pyrido
II: Pyrido

4. Verbindung nach einem der Ansprüche 1 bis 3,
dadurch **gekennzeichnet**, daß an mindestens einem Kern des Heteroaromaten in Orthostellung zum N-Atom ein $CH_3$-Rest vorgesehen ist.

5. Verbindung nach Anspruch 4,
dadurch **gekennzeichnet**, daß der $CH_3$-Rest beispielsweise durch Kondensation mit para-Methoxybenzaldehyd substituiert ist.

## Claims

1. Compound of the general formula

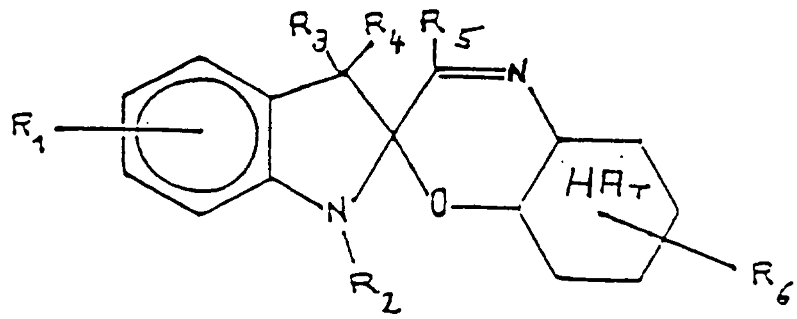

where the symbols mean:

| | |
|---|---|
| $R_1$ | one to four substitute groups from the series -Z, -OZ, $-NZ_2$, -NHZ, $-NH_2$, -CN, $CF_3$, where Z: $C_1$-$C_5$ alkyl, phenyl, pyridyl, benzyl |
| $R_2$ | -H, -Z, $-CH_2Z$ |
| $R_3$,$R_4$,$R_5$ | $-NH_2$, NHZ, $-NZ_2$, -OZ, -Z, -H |
| $R_6$ | -H, -Z, -OZ, -CN, $NO_2$ |
| HAr | two or three-nucleus heteromatic substances with 1 or 2 N atoms in accordance with one of the following structure formulae |

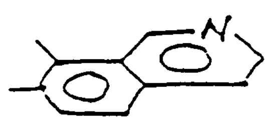

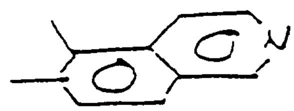

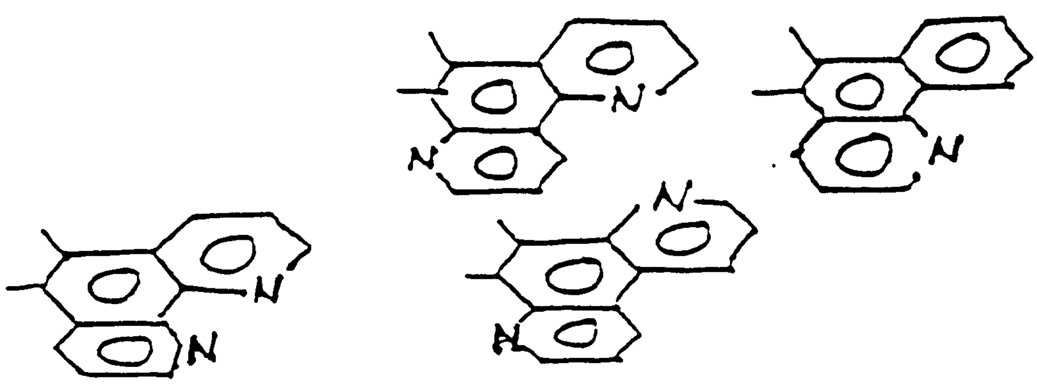

2. Compound of the general formula given in Claim 1 where

$R_1$: H
$R_2$: $CH_3$
$R_3.R_4$: $CH_3$ or $C_2H_5$
$R_5$: H

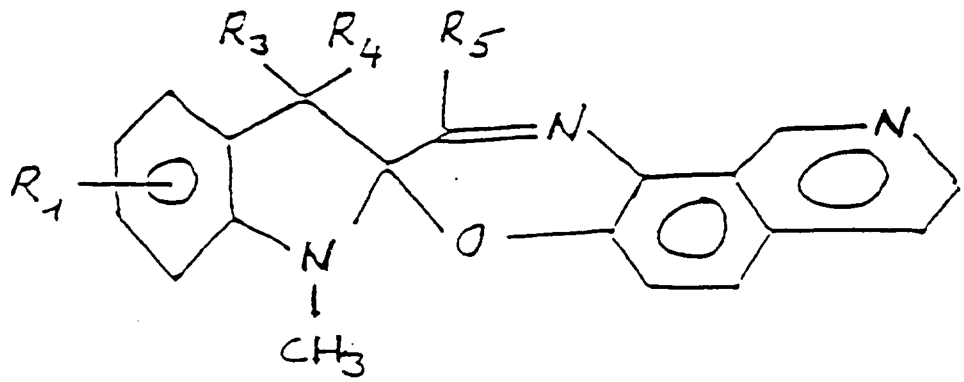

3. Compound of the general formula in accordance with Claim 1 where

$R_1$, $R_5$: H,
$R_2$, $R_3$, $R_4$: $CH_3$

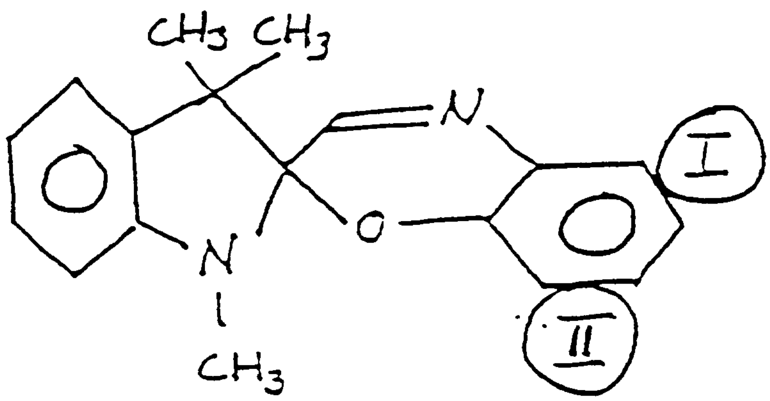

I: benzo or pyrido
II: Pyrido.

4. Compound according to any of claims 1 to 3,
characterised by the fact that at least one nucleus of the heteroaromatic substance is provided with a $CH_3$ residue in an ortho-position to the N atom.

5. Compound according to claim 4,
characterised by the fact that the $CH_3$ residue is substituted, for example by condensation, by para-methoxybenzaldehyde

## Revendications

1. Composé répondant à la formule générale

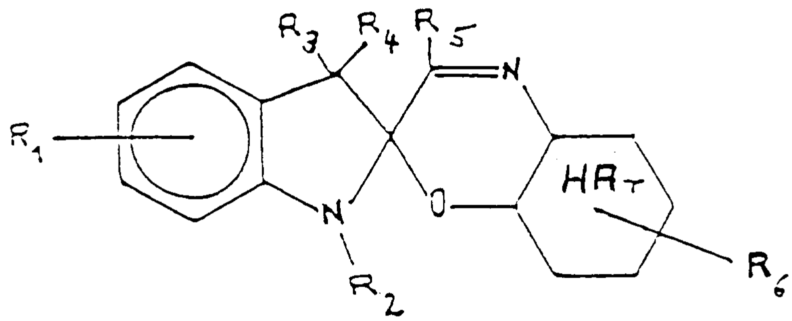

dans laquelle :

$R_1$ représente 1 à 4 substituants choisis dans la série formée par un groupe -Z, -OZ, $-NZ_2$, -NHZ, $-NH_2$, -CN, $-CF_3$,
où Z représente un groupe alkyle en $C_1$ à $C_5$, un groupe phényle, pyridyle, benzyle,
$R_2$ représente -H, un groupe -Z, $-CH_2Z$,
$R_3$, $R_4$ et $R_5$ représentent chacun un groupe $-NH_2$, -NHZ, $-NZ_2$, -OZ, -Z, -H
$R_6$ représente -H, un groupe -Z, -OZ, -CN, $-NO_2$,
HAr représente un reste hétéro-aromatique comportant deux ou trois noyaux (bicycliques ou tricycliques) et ayant 1 ou 2 atomes d'azote, selon l'une des formules de structure ci-après :

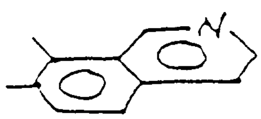

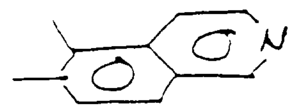

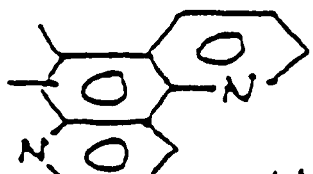

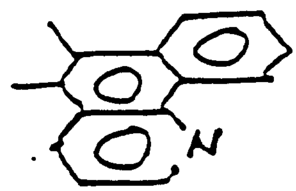

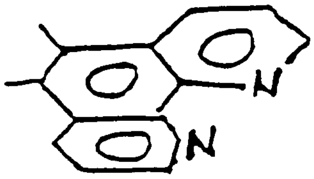

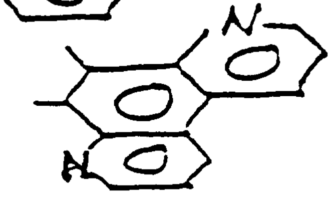

2. Composé répondant à la formule générale indiquée à la revendication 1, dans laquelle

$R_1$ représente H,
$R_2$ un groupe $CH_3$
$R_3$ et $R_4$ représentent chacun un groupe $CH_3$ ou $C_2H_5$,
$R_5$ représente H,

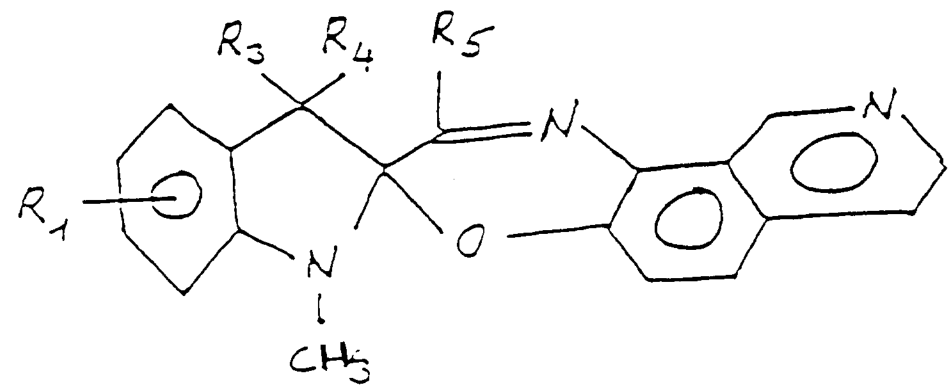

3. Composé de formule générale selon la revendication 1, dans laquelle :

$R_1$ et $R_5$ représentent chacun H,
$R_2$, $R_3$ et $R_4$ représentent chacun $CH_3$

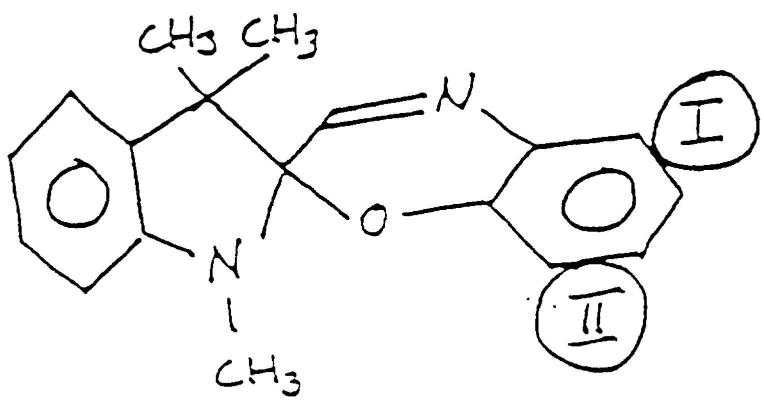

où
I : benzo ou pyrido
II : pyrido.

4. Composé selon l'une des revendications 1 à 3, caractérisé en ce qu'un reste $CH_3$ est prévu en position ortho par rapport à l'atome d'azote dans au moins un noyau du composé ou fragment hétéro-aromatique.

5. Composé selon la revendication 4, caractérisé en ce que le reste $CH_3$ porte un substituant, fixé par exemple par condensation avec le para-méthoxybenzaldéhyde.